# EUROPEAN PATENT SPECIFICATION

(11) **EP 4 479 118 B1**
(45) Date of publication and mention of the grant of the patent: **04.11.2026**
(21) Application number: 23712680.0
(22) Date of filing: 01.03.2023
(51) Int. Cl.: A61M 25/09, A61M 25/01, A61M 25/00, A61B 17/22

(54) **GUIDEWIRES AND ASSEMBLIES FOR AUTOMATIC WITHDRAWAL OF STYLETS FROM CATHETERS**
FÜHRUNGSDRÄHTE UND VORRICHTUNGEN ZUM AUTOMATISCHEN HERAUSZIEHEN VON STILETTS AUS KATHETERN
FILS-GUIDES ET ASSEMBLAGES POUR LE RETRAIT AUTOMATIQUE DES STYLETS DES CATHÉTERS

(30) Priority: 02.03.2022 US 202263315903 P
(43) Date of publication of application: 25.12.2024
(73) Proprietor: Bard Access Systems, Inc., Salt Lake City, UT 84116 (US)
(72) Inventor: HOWELL, Glade, H., Draper, UT 84020 (US); THORNLEY, Kyle, G., Farmington, UT 84025 (US)
(74) Representative: Hoffmann Eitle
(86) International application number: PCT/US2023/014295
(87) International publication number: WO 2023/167940

(56) References cited:
- WO-A1-2008/133808
- US-A- 5 059 183
- US-A1- 2008 058 839
- US-A1- 2012 071 857
- US-A1- 2021 361 915

## Description

### PRIORITY

This application claims the benefit of priority to U.S. Provisional Patent Application No. 63/315,903, filed March 2, 2022.

### BACKGROUND

Insertion of central venous catheters ("CVCs") into patients is a multi-step process subject to multiple occasions for error. For example, after percutaneously puncturing a patient with a needle at a chosen insertion site, whereby a needle tract is established from an area of skin to a blood-vessel lumen, an access guidewire is inserted through the needle into the blood-vessel lumen. After the needle is removed, a CVC is inserted into the blood-vessel lumen over the access guidewire. At this point, the access guidewire can be exchanged with a maneuver guidewire over which a catheter tube of the CVC is subsequently advanced through a vasculature of the patient. However, the CVC can include a stylet as assembled during manufacturing, which stylet fills excess annular space between an inner diameter of the catheter tube and an outer diameter of the access guidewire that otherwise causes a tip of the catheter tube to catch on the skin of the patient as the CVC is inserted. In addition, the stylet stiffens and provides additional column strength to the catheter tube for preventing buckling when the CVC is inserted into the blood-vessel lumen over the access guidewire. While this stylet should be removed after the CVC is inserted into the blood-vessel lumen over the access guidewire, the stylet is often forgotten, which can lead to procedural inefficacy and even patient trauma. In view of the foregoing, an automatic stylet-removing means and method for removing stylets from CVCs or other catheters is needed.

Disclosed herein are guidewires, stylet assemblies, catheter assemblies, and methods thereof that address at least the foregoing. The disclosed methods do not form part of the invention.

WO 2008/133808 A1 discloses a medical guidewire system comprising a first inner member having a first outer diameter, a second intermediate member having a second outer diameter larger than the first outer diameter, and a third outer member having a third diameter larger than the second outer diameter of the second member. The second member has a longitudinal extending opening to receive the first member for sliding movement with respect to the first member and the third outer member has a longitudinally extending opening to receive the second member for sliding movement with respect to the first and second member. The first member has a first stiffness, the third member has a third stiffness greater than the first stiffness, and the second member is movable to stiffen the guidewire system.

US 2008/058839 A1 discloses a reverse tapered guidewire and method of use.

US 2012/071857 A1 discloses a method and apparatus for treating disorders of the sinuses.

US 2021/0361915 A1 discloses rapidly insertable central catheters including catheter assemblies and methods thereof.

US5059183 A1 discloses a guidewire with an enlarged, atraumatic J-shaped tip.

### SUMMARY

In a first aspect of the present invention, there is provided a stylet assembly according to claim 1. In a second aspect of the present invention, there is provided a stylet assembly according to claim 2. The dependent claims define preferred embodiments.

Disclosed herein is a guidewire including, in some embodiments, a proximal portion, a distal portion, and a guidewire tip in the distal portion of the guidewire. The proximal portion has a first outer diameter. The distal portion includes a dilated section having a second outer diameter greater than the first outer diameter. The dilated section of the guidewire is configured to butt against a stylet tip of a stylet when at least the proximal portion of the guidewire is disposed therein with a corresponding orientation and withdrawn therefrom in a proximal direction by the proximal portion of the guidewire.

In some embodiments, the guidewire includes a step delimiting the dilated section with the second outer diameter from a remainder of the guidewire with the first outer diameter.

In some embodiments, the guidewire includes a taper from the dilated section with the second outer diameter to a remainder of the guidewire with the first outer diameter.

In some embodiments, the guidewire tip is an atraumatic 'J'-shaped guidewire tip.

Also disclosed herein is a guidewire including, in some embodiments, a proximal portion, a distal portion, and a guidewire tip in the distal portion of the guidewire. The proximal and distal portions of the guidewire have a first outer diameter. The guidewire tip in the distal portion of the guidewire terminates with a spherical end cap. The spherical end cap has a third diameter greater than the first outer diameter such that the spherical end cap butts against a stylet tip of a stylet when at least the proximal portion of the guidewire is disposed therein with a corresponding orientation and withdrawn therefrom in a proximal direction by the proximal portion of the guidewire.

Also disclosed herein is a stylet assembly including, in some embodiments, a guidewire disposed in a stylet. The guidewire includes a proximal portion, a distal portion, and a guidewire tip in the distal portion of the guidewire. The proximal portion has a first outer diameter. The distal portion includes a dilated section having a second outer diameter greater than the first outer diameter. The stylet includes a stylet tip and a stylet lumen therethrough. The stylet lumen has a stylet inner diameter commensurate with the first outer diameter of the guidewire. At least the proximal portion of the guidewire is disposed in the stylet lumen of the stylet with a corresponding orientation. The dilated section of the guidewire is configured to butt against the stylet tip when the guidewire is proximally withdrawn from a catheter for automatically withdrawing the stylet from the catheter with the guidewire.

In some embodiments, the guidewire includes a step delimiting the dilated section with the second outer diameter from a remainder of the guidewire with the first outer diameter.

In some embodiments, the guidewire includes a taper from the dilated section with the second outer diameter to a remainder of the guidewire with the first outer diameter.

According to the invention, the guidewire tip is an atraumatic 'J'-shaped guidewire tip.

Also disclosed herein is a catheter assembly including, in some embodiments, a guidewire disposed in a stylet and the stylet disposed in a catheter. The guidewire includes a proximal portion, a distal portion, and a guidewire tip in the distal portion of the guidewire. The proximal portion has a first outer diameter. The distal portion includes a dilated section having a second outer diameter greater than the first outer diameter. The stylet includes a stylet tip and a stylet lumen therethrough. The stylet lumen has a stylet inner diameter commensurate with the first outer diameter of the guidewire. The catheter includes a catheter tube including a catheter tip and a primary lumen therethrough. The primary lumen has a catheter inner diameter commensurate with a stylet outer diameter within at least the catheter tube. At least the proximal portion of the guidewire is disposed in the stylet lumen of the stylet with a corresponding orientation, and the stylet is disposed in the primary lumen of the catheter with a corresponding orientation. The dilated section of the guidewire is configured to butt against the stylet tip when the guidewire is proximally withdrawn from the catheter for automatically withdrawing the stylet from the catheter with the guidewire.

In some embodiments, the guidewire includes a step delimiting the dilated section with the second outer diameter from a remainder of the guidewire with the first outer diameter.

In some embodiments, the guidewire includes a taper from the dilated section with the second outer diameter to a remainder of the guidewire with the first outer diameter.

According to the invention, the guidewire tip is an atraumatic 'J'-shaped guidewire tip.

In some embodiments, the catheter is a triluminal catheter including a trifurcated catheter hub coupled to a proximal portion of the catheter tube and three extension legs extending from the catheter hub. Each extension leg of the three extension legs includes a Luer connector coupled to a proximal portion of the extension leg.

In some embodiments, the primary lumen extends from an opening in a proximal end of a first Luer connector to an opening in the catheter tip, a secondary lumen extends from an opening in a proximal end of a second Luer connector to a secondary-lumen opening in a distal portion of the catheter tube, and a tertiary lumen extends from an opening in a proximal end of a third Luer connector to a tertiary-lumen opening in the distal portion of the catheter tube proximal of the secondary-lumen opening.

Also disclosed herein for illustrative purpose is a non-claimed method of inserting a catheter into a blood-vessel lumen of a patient. The method includes, in some embodiments, a needle tract-establishing step, a guidewire-inserting step, a needle-withdrawing step, a catheter-advancing step, and an automatic stylet-withdrawing step. The needle tract-establishing step includes establishing a needle tract from an area of skin to the blood-vessel lumen with a needle. The guidewire-inserting step includes inserting a guidewire into a needle lumen of the needle and advancing the guidewire into the blood-vessel lumen. The needle-withdrawing step includes withdrawing the needle from the needle tract leaving the guidewire in place in the blood-vessel lumen. The catheter-advancing step includes advancing a catheter including a stylet disposed in a primary lumen thereof over the guidewire with at least a proximal portion of the guidewire disposed in a stylet lumen of the stylet until a sufficient portion of a catheter tube of the catheter is disposed in the blood-vessel lumen. The automatic stylet-withdrawing step includes withdrawing both the guidewire and the stylet from the catheter. The guidewire includes the proximal portion having a first outer diameter and a distal portion including a dilated section having a second outer diameter greater than the first outer diameter. The dilated section of the guidewire is configured to butt against a stylet tip of the stylet when the guidewire is proximally withdrawn from the catheter for automatically withdrawing the stylet from the catheter with the guidewire.

In some embodiments, the guidewire includes a step delimiting the dilated section with the second outer diameter from a remainder of the guidewire with the first outer diameter.

In some embodiments, the guidewire includes a taper from the dilated section with the second outer diameter to a remainder of the guidewire with the first outer diameter.

In some embodiments, the guidewire tip is an atraumatic 'J'-shaped guidewire tip.

In some embodiments, the primary lumen of the catheter has a catheter inner diameter commensurate with a stylet outer diameter within at least the catheter tube of the catheter such that annular space otherwise therebetween does not cause a catheter tip of the catheter tube to catch on skin of the patient during the advancing of the catheter into the blood-vessel lumen of the patient.

These and other features of the concepts provided herein will become more apparent to those of skill in the art in view of the accompanying drawings and following description, which describe particular embodiments of such concepts in greater detail.

### DRAWINGS

FIG. 1 illustrates a catheter assembly including a catheter, a stylet, and a guidewire for automatically withdrawing the stylet from the catheter in accordance with some embodiments.
FIG. 2 illustrates a detailed view of a distal portion of the guidewire including a dilated section before butting against a stylet tip of the stylet as the guidewire is withdrawn in a proximal direction from the catheter in accordance with some embodiments.
FIG. 3 illustrates a detailed view of the dilated section of the guidewire while butting against the stylet tip of the stylet as the guidewire is withdrawn in the proximal direction from the catheter in accordance with some embodiments.
FIG. 4 illustrates a detailed view of the dilated section of the guidewire while butting against the stylet tip of the stylet just before the stylet is automatically withdrawn from the catheter by way of the guidewire as the guidewire is withdrawn in the proximal direction from the catheter in accordance with some embodiments.
FIG. 5 illustrates a detailed view of the dilated section of the guidewire while the stylet continues to be automatically withdrawn from the catheter by way of the guidewire as the guidewire is withdrawn in the proximal direction from the catheter in accordance with some embodiments.
FIG. 6 illustrates a detailed view of the distal portion of the guidewire including a spherical end cap instead of the dilated section of the guidewire before the spherical end cap butts against the stylet tip of the stylet as the guidewire is withdrawn in a proximal direction from the catheter in accordance with some embodiments.
FIG. 7 illustrates another detailed view of the distal portion of the guidewire including the spherical end cap before butting against the stylet tip of the stylet as the guidewire is withdrawn in a proximal direction from the catheter in accordance with some embodiments.
FIG. 8 illustrates a detailed view of the spherical end cap of the guidewire while butting against the stylet tip of the stylet just before the stylet is automatically withdrawn from the catheter by way of the guidewire as the guidewire is withdrawn in the proximal direction from the catheter in accordance with some embodiments.
FIG. 9 illustrates a detailed view of the spherical end cap of the guidewire while butting against the stylet tip of the stylet as the stylet is automatically withdrawn from the catheter by way of the guidewire as the guidewire is withdrawn in the proximal direction from the catheter in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein, as long as such modification falls under the scope of the appended claims.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. In addition, any of the foregoing features or steps can, in turn, further include one or more features or steps unless indicated otherwise. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or "proximal section" of, for example, a catheter includes a portion or section of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal section, or the proximal length of the catheter can include the proximal end of the catheter; however, the proximal portion, the proximal section, or the proximal length of the catheter need not include the proximal end of the catheter. That is, unless context suggests otherwise, the proximal portion, the proximal section, or the proximal length of the catheter is not a terminal portion or terminal length of the catheter.

With respect to "distal," a "distal portion" or a "distal section" of, for example, a catheter includes a portion or section of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal section, or the distal length of the catheter can include the distal end of the catheter; however, the distal portion, the distal section, or the distal length of the catheter need not include the distal end of the catheter. That is, unless context suggests otherwise, the distal portion, the distal section, or the distal length of the catheter is not a terminal portion or terminal length of the catheter.

Unless defined otherwise, all technical and scientific terms used herein have the same meaning as commonly understood by those of ordinary skill in the art.

As set forth above, insertion of CVCs into patients is a multi-step process subject to multiple occasions for error. For example, after percutaneously puncturing a patient with a needle at a chosen insertion site, whereby a needle tract is established from an area of skin to a blood-vessel lumen, an access guidewire is inserted through the needle into the blood-vessel lumen. After the needle is removed, a CVC is inserted into the blood-vessel lumen over the access guidewire. At this point, the access guidewire can be exchanged with a maneuver guidewire over which a catheter tube of the CVC is subsequently advanced through a vasculature of the patient. However, the CVC can include a stylet as assembled during manufacturing, which stylet fills excess annular space between an inner diameter of the catheter tube and an outer diameter of the access guidewire that otherwise causes a tip of the catheter tube to catch on the skin of the patient as the CVC is inserted. In addition, the stylet stiffens and provides additional column strength to the catheter tube for preventing buckling when the CVC is inserted into the blood-vessel lumen over the access guidewire. While this stylet should be removed after the CVC is inserted into the blood-vessel lumen over the access guidewire, the stylet is often forgotten, which can lead to procedural inefficacy and even patient trauma. In view of the foregoing, an automatic stylet-removing means and method for removing stylets from CVCs or other catheters is needed.

Disclosed herein are guidewires, stylet assemblies, catheter assemblies, and methods thereof that address at least the foregoing. The disclosed methods do not form part of the invention. A stylet assembly includes a guidewire disposed in a stylet. The guidewire includes a proximal portion having a first outer diameter, a distal portion including a dilated section having a second outer diameter greater than the first outer diameter, and a guidewire tip in the distal portion of the guidewire. The stylet includes a stylet tip and a stylet lumen therethrough with a stylet inner diameter commensurate with the first outer diameter of the guidewire. At least the proximal portion of the guidewire is disposed in the stylet lumen of the stylet with a corresponding orientation. The dilated section of the guidewire is configured to butt against the stylet tip when the guidewire is proximally withdrawn from a catheter for automatically withdrawing the stylet from the catheter with the guidewire. The guidewire tip is an atraumatic 'J'-shaped guidewire tip.

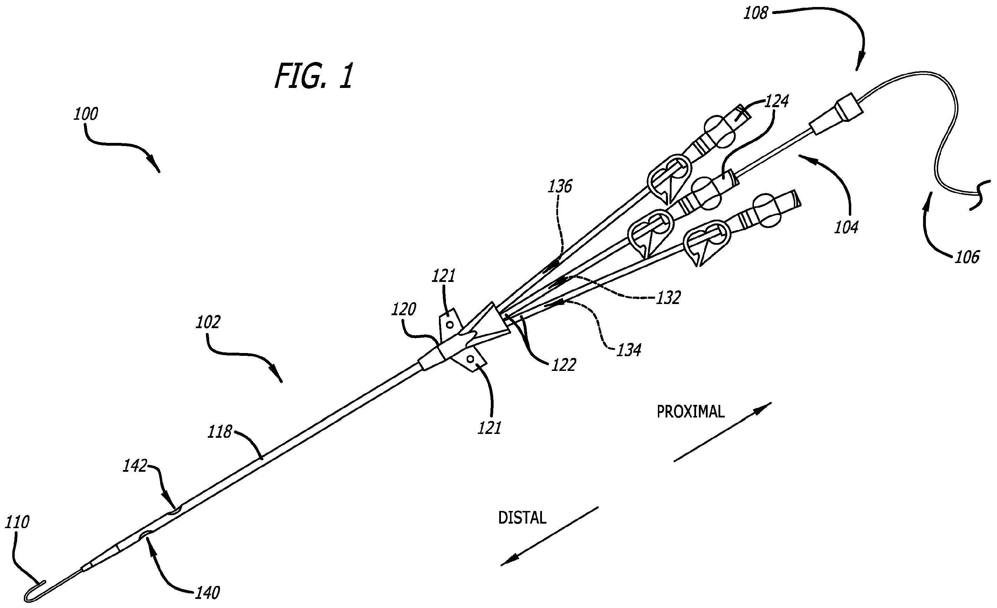

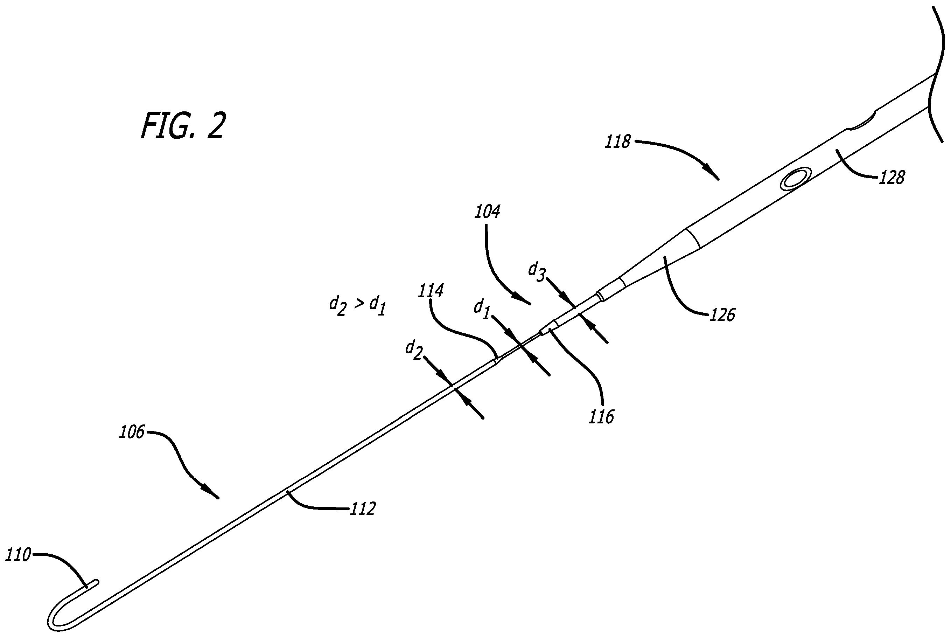

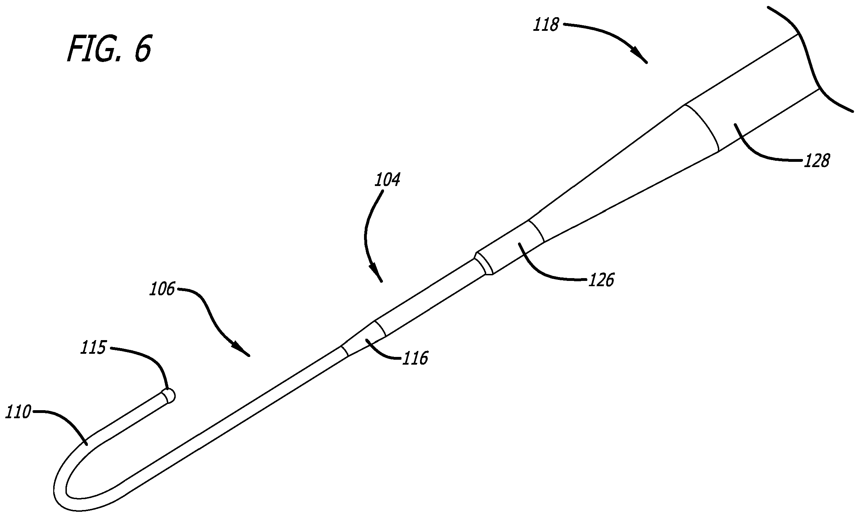

FIG. 1 illustrates a catheter assembly 100 including a catheter 102, a stylet 104, and a guidewire 106 for automatically withdrawing the stylet 104 from the catheter 102 in accordance with some embodiments. FIGS. 2-5 and 6-9 illustrate various detailed views of a distal portion of the catheter assembly 100 in accordance with some embodiments. Notably, FIGS. 2-5 illustrate the catheter assembly 100 with the guidewire 106 having the dilated section 112 thereof, while FIGS. 6-9 illustrate the catheter assembly 100 with the guidewire 106 having the spherical end cap 115 of the guidewire tip 110, thereby illustrating the guidewire 106 with two different features or automatic stylet-removing means for automatically removing the stylet 104 from the catheter assembly 100.

As shown, the catheter assembly 100 includes the guidewire 106 disposed in the stylet 104 (e.g., the stylet lumen of the stylet 104), thereby forming a stylet assembly 108, and the stylet assembly 108 disposed in the catheter 102 (e.g., the primary lumen 132 of the catheter 102) with corresponding orientations (i.e., distal portions or distal ends thereof of the guidewire 106, the stylet 104, and the catheter 102 are distally oriented while proximal portions of proximal ends thereof of the guidewire 106, the stylet 104, and the catheter 102 are proximally oriented).

The guidewire 106 (e.g., an access guidewire) includes a proximal portion, a distal portion, and a guidewire tip 110 in the distal portion of the guidewire 106.

With reference to the guidewire 106 of FIGS. 2-5, the proximal portion of the guidewire 106 has an outer diameter, nominally a first outer diameter as used herein. *(See* diameter *d₁* in FIG. 2 with the understanding the proximal and distal portions of the guidewire 106 both have the first outer diameter proximal of the dilated section 112 in the embodiment illustrated in FIGS. 2-5.) The first outer diameter of the guidewire 106 can be constant along a length of the proximal portion of the guidewire 106.

Further with reference to the guidewire 106 of FIGS. 2-5, the distal portion of the guidewire 106 includes a dilated section 112 having an outer diameter, nominally a second outer diameter as used herein. (*See* diameter *d₂* in FIG. 2.) The second outer diameter of the guidewire 106, specifically, that of the dilated section 112 of the guidewire 106, is greater than the first outer diameter of the guidewire 106 (i.e., *d₂ > d₁*) whether the first outer diameter is in the proximal portion of the guidewire 106 or the distal portion of the guidewire 106 proximal of the dilated section 112. (*See* FIG. 2, where *d₁* corresponds to the first outer diameter of the distal portion of the guidewire 106 proximal of the dilated section 112 of the guidewire 106.) The second outer diameter of the guidewire 106 can be constant along a length of the dilated section 112 of the guidewire 106; however, the second outer diameter can have the taper 114 set forth below. Notwithstanding the foregoing, the dilated section 112 of the guidewire 106, for example, a proximal portion or proximal end of the dilated section 112 of the guidewire 106, is configured to butt against the stylet tip 116 of the stylet 104 when the guidewire 106 is proximally withdrawn from the catheter 102 for automatically withdrawing the stylet 104 from the catheter 102 with the guidewire 106 as the stylet assembly 108.

Further with reference to the guidewire 106 of FIGS. 2-5, the dilated section 112 of the guidewire 106 can have a taper 114 that tapers from the second outer diameter of the dilated section 112 to the first outer diameter of a remainder of the guidewire 106 proximal of the dilated section 112 such as the distal portion of the guidewire 106 proximal of the dilated section 112 as best shown in FIG. 2 or the proximal portion of the guidewire 106. The taper 114 can be relatively short (e.g., ≤ 5% the length of the dilated section 112 of the guidewire 106 such as ≤ 3%, 2%, or 1% the length of the dilated section 112 of the guidewire 106) as shown in FIG. 2 or relatively long (e.g., ≥ 25% the length of the dilated section 112 of the guidewire 106). Such a taper can be established with the windings of the one-or-more round wires set forth below when, for example, the one-or-more round wires are individually tapered or ground down to form the taper 114. Alternatively, the guidewire 106 can include a step delimiting the dilated section 112 with the second outer diameter from the remainder of the guidewire 106 with the first outer diameter.

With reference to the guidewire 106 of FIGS. 6-9, both the proximal and distal portions of the guidewire 106 can have the first outer diameter set forth above, which can be constant along a length of the guidewire 106 except for that associated with the spherical end cap 115. That said, the proximal and distal portions of the guidewire 106 can have different outer diameters if the proximal and distal portions of the guidewire 106 differ in their construction as set forth below with respect to the one-or-more core wires and different options for any windings therearound. But any different outer diameters of the proximal and distal portions of the guidewire 106 of FIGS. 6-9 that might result from their different constructions should be understood as not affecting the spherical end cap 115 as the automatic stylet-removing means for automatically removing the stylet 104 from the catheter assembly 100 of FIGS. 6-9.

The guidewire tip 110 being an atraumatic 'J'-shaped guidewire tip as best shown in FIGS. 2-5 and FIG. 6. Such a 'J'-shaped guidewire tip can terminate with a hemispherical end cap as shown in FIGS. 2-5; however, such a 'J'-shaped guidewire tip can alternatively terminate with a spherical end cap 115. In such embodiments, the spherical end cap 115 of the guidewire tip 110 has a diameter, nominally a third diameter as used herein, which is at least commensurate with the second outer diameter of the dilated section 112 set forth above and, therefore, greater than the first outer diameter of the guidewire 106. The spherical end cap 115 obviates the dilated section 112 of the guidewire 106 illustrated in FIGS 2-5 such that the proximal and distal portions of the guidewire 106 can both have the first outer diameter. And like the dilated section 112 of the guidewire 106 of FIGS. 2-5, the spherical end cap 115 of the guidewire tip 110 illustrated in FIGS. 6-9 is likewise configured to butt against the stylet tip 116 of the stylet 104 when the guidewire 106 is proximally withdrawn from the catheter 102 for automatically withdrawing the stylet 104 from the catheter 102 with the guidewire 106 as the stylet assembly 108.

The guidewire 106 can be one or more bare core wires, round-wound with windings of one or more round wires around the one-or-more core wires, ground-wound with the windings of the one-or-more round wires ground down to flatten the windings, flat-wound with the windings of one or more tapes instead of the one-or-more round wires, or a portionwise combination thereof. In an example, the guidewire 106 can include at least a bare-wire portion and a round-wound portion with the round-wound portion of the guidewire 106 distal of the bare-wire portion of the guidewire 106, the round-wound portion of the guidewire 106 corresponding to the dilated section 112 of the guidewire 106. In another example, the guidewire 106 can include at least a ground-wound or flat-wound portion and a round-wound portion with the round-wound portion of the guidewire 106 distal of the ground-wound or flat-wound portion of the guidewire 106, the round-wound portion of the guidewire 106 corresponding to the dilated section 112 of the guidewire 106. Should any guidewire of the foregoing example guidewires be assembled into a sealed needle assembly for both establishing a percutaneous puncture and inserting the guidewire 106 into a blood-vessel lumen via a needle tract established by the needle assembly, the bare-wire, ground-wound, or flat-wound portion of the guidewire 106 having fewer gaps than the round-wound portion can advantageously facilitate a seal of the sealed needle assembly in establishing a leak-free seal around the guidewire 106. Each core wire of the one-or-more core wires can be selected from a round or cylindrical core wire, a prismatic core wire such as a core wire of an extended triagonal, tetragonal, pentagonal, hexagonal, heptagonal, or octagonal prism, and portionwise combinations thereof. Whether the guidewire 106 is round-wound, ground-wound, or flat-wound, edges of any prismatic core wire present in the guidewire 106 can advantageously bite into the one-or-more round wires or tapes, thereby minimizing slippage of the windings over the one or more core wires. Notably, stiffness of the guidewire 106 or portions thereof can be tuned by way of the one-or-more core wires with the one-or-more core wires or portions thereof having more bite producing stiffer guidewires or portions of the guidewire 106.

As shown, the stylet 104 includes a stylet tip 116 and a stylet lumen therethrough.

The stylet lumen has a stylet inner diameter commensurate with the first outer diameter of the guidewire 106.

The stylet 104 also includes a stylet outer diameter (*see,* for example, diameter *d₃* in FIG. 2) commensurate with a catheter inner diameter of the primary lumen 132 of the catheter 102 set forth below, particularly within at least the catheter tube 118 of the catheter 102. By way of the stylet outer diameter, the stylet 104 occupies annular space that would otherwise be between the first outer diameter of the guidewire 106 and the catheter inner diameter of the primary lumen 132 of the catheter 102. When not occupied, such annular space can cause the catheter tip 130 of the catheter tube 118 to catch on skin of a patient when advancing the catheter 102 into a blood-vessel lumen of the patient over the guidewire 106.

As shown, the catheter 102 includes a catheter tube 118, a catheter hub 120, one or more extension legs 122, and one or more extension-leg connectors 124. Such a catheter can be a rapidly insertable central catheter ("RICC"); however, the catheter 102 is not limited thereto.

The catheter tube 118 includes a first section 126 in a distal portion of the catheter tube 118 and a second section 128 proximal of the first section 126.

The first section 126 of the catheter tube 118 distally terminates with a catheter tip 130. In addition, the first section 126 of the catheter tube 118 is tapered from a flat-faced proximal end of the first section 126, which proximal end is fixedly coupled to the distal end of the second section 128 of the catheter tube 118, to a distal end of the first section 126 or the catheter tip 130 thereof. Indeed, the first section 126 of the catheter tube 118 includes a taper from a catheter outer diameter of the proximal end of the first section 126, which catheter outer diameter is commensurate with the catheter outer diameter of the second section 128 of the catheter tube 118, to a catheter outer diameter of the distal end of the first section 126 or the catheter tip 130 thereof. The taper of the first section 126 of the catheter tube 118 is configured for immediate dilation of tissue about a needle tract established with an introducer needle up to the catheter outer diameter of the second section 128 of the catheter tube 118.

The second section 128 of the catheter tube 118 includes a consistent catheter outer diameter over its length from a flat-faced distal end of the second section 128 to a proximal end of the second section 128. The consistent diameter of the second section 128 of the catheter tube 118 is configured for smooth insertion into the foregoing needle tract and targeted vasculature subsequent to any dilation by the first section 126 of the catheter tube 118.

The first and second sections 126 and 128 of the catheter tube 118 are fixedly coupled together at their ends. Indeed, the flat-faced proximal end of the first section 126 of the catheter tube 118 is bonded to the flat-faced distal end of the second section 128 of the catheter tube 118 by a solvent bond, an adhesive bond, or a heat weld. In addition, an abluminal surface of the catheter tube 118 smoothly transitions from an abluminal surface of the first section 126 of the catheter tube 118 to an abluminal surface of the second section 128 of the catheter tube 118 with minimal to negligible edges that do not catch on skin when the catheter tube 118 is inserted into a needle tract. And when solvent bonded, the minimal to negligible edges can include solvent-interdiffused polymeric material of the polymeric materials from which the first and second sections 126 and 128 of the catheter tube 118 are formed, which smoothens the transition from the first section 126 of the catheter tube 118 to the second section 128 of the catheter tube 118.

The first section 126 of the catheter tube 118 can be formed of a first polymeric material (e.g., a polytetrafluoroethylene, a polypropylene, or a polyurethane) having a first durometer. The second section 128 of the catheter tube 118 can be formed of a second polymeric material (e.g., a polyvinyl chloride, a polyethylene, another polyurethane, or a silicone) having a second durometer less than the first durometer. For example, the first section 126 of the catheter tube 118 can be formed of a first polyurethane having the first durometer while the second section 128 of the catheter tube 118 can be formed of a second, different polyurethane (e.g., a same or different diisocyanate or triisocyanate reacted with a different diol or triol, a different diisocyanate or triisocyanate reacted with a same or different diol or triol, a same diisocyanate or triisocyanate reacted with a same diol or triol under different conditions or with different additives, etc.) having the second durometer less than the first durometer. Indeed, polyurethanes are advantageous for the catheter tube 118 in that polyurethanes can be relatively rigid at room-temperature but become more flexible *in vivo* at body temperature, which reduces irritation to vessel walls as well as phlebitis. Polyurethanes are also advantageous in that they can be less thrombogenic than some other polymers. Notwithstanding the foregoing, however, the first and second sections 126 and 128 of the catheter tube 118 can be formed of a same polymeric material selected from the foregoing polymeric materials.

It should be understood the first durometer of the first polymeric material and the second durometer of the second polymeric material can be on different scales (e.g., Type A or Type D). With this understanding, the second durometer of the second polymeric material might not be numerically less than the first durometer of the first polymeric material when the second durometer is less than the first durometer. Indeed, the hardness of the second polymeric material can still be less than the hardness of the first polymeric material as the different scales-each of which ranges from 0 to 100-are designed for characterizing different materials in groups of the materials having a like hardness.

Adverting back to the catheter tip 130 of the first section 126 of the catheter tube 118, the catheter tip 130 can include a reinforcement band as a reinforcer within the catheter tip 130. Such a reinforcement band is sufficiently rigid over its length to support aspiration through the catheter tube 118 without collapsing the catheter tip 130. Additionally or alternatively, the reinforcement band is sufficiently rigid over its length to prevent buckling of the catheter tip 130 when the catheter tube 118 is rapidly inserted into a needle tract to a blood-vessel lumen of a target vasculature over the guidewire 106. The reinforcement band can be formed of a metal or a thermoplastic polymer inserted into a bore of the first section 126 of the catheter tube 118 commensurate with the reinforcement band. Alternatively, the first section 126 of the catheter tube 118 can be molded or extruded over the metallic or polymeric reinforcement band. When the reinforcement band is formed of the metal, the metal can be selected from at least stainless steel, titanium, and nitinol. When the reinforcement band is formed of the thermoplastic polymer, the thermoplastic polymer can be selected from at least a thermoplastic polyurethane, a carbonate-based thermoplastic polyurethane, a polyamide, and a polyamide-block-polyether copolymer. Advantageously, the thermoplastic polymer of the reinforcement band can be selected to remain relatively rigid *in vivo* at body temperature in contrast to that set forth above for the first and second sections 126 and 128 of the catheter tube 118. In addition, the thermoplastic polymer of the reinforcement band can be formulated to include a radiopacifier such as bismuth trioxide or barium sulfate to enhance radiopacity of the catheter tip 130.

The catheter tube 118 or also includes one or more catheter-tube lumens extending through the catheter tube 118; however, only one catheter-tube lumen typically extends from a proximal end of the catheter tube 118 to a distal end of the catheter tube 118 in a monoluminal or multiluminal catheter (e.g., a diluminal catheter, a triluminal catheter, a tetraluminal catheter, a pentaluminal catheter, a hexaluminal catheter, etc.). Indeed, the first section 126 of the catheter tube 118 typically includes a single lumen therethrough, namely the primary lumen 132. Notably, each lumen of the secondary lumen 134 and the tertiary lumen 136 terminates at the distal end of the second section 128 of the catheter tube 118.

In accordance with the first section 126 of the catheter tube 118, the second section 128 of the catheter tube 118, and the catheter tip 130 set forth above, the catheter tube 118 possesses a column strength, optionally, in combination with the guidewire 106 disposed therein, sufficient to prevent buckling of the catheter tube 118 or when rapidly inserted into a needle tract to a blood-vessel lumen of a patient. The column strength of the catheter tube 118 is also sufficient to prevent buckling of the catheter tube 118 when advanced through a vasculature of the patient without dilation of tissue about the needle tract or any blood vessels of the vasculature beforehand with a separate dilator. Indeed, the column strength of the catheter tube 118 is notable in that the column strength makes it possible to rapidly insert the catheter 102 or the catheter tube 118 thereof into a needle tract to a blood-vessel lumen and advance the catheter tube 118 through the vasculature of the patient without using the Seldinger technique.

The catheter hub 120 includes a pair of suture wings 121 including a number of suture-wing through holes for suturing the catheter hub 120 to a patient. Each wing of the pair of suture wings 121 can include one, two, three, or four suture-wing through holes for suturing the catheter hub 120 to the patient.

The catheter hub 120 also includes one or more catheter-hub lumens corresponding in number to the one-or-more catheter-tube lumens. The one-or-more catheter-hub lumens extends through an entirety of the catheter hub 120 from a proximal end of the catheter hub 120 to a distal end of the catheter hub 120. When the catheter hub 120 is coupled to a proximal portion of the catheter tube 118 as shown in FIG. 1, the one-or-more catheter-tube lumens continue through the one-or-more catheter-hub lumens, respectively.

Each extension leg of the one-or-more extension legs 122 is coupled to-and, therefore, extends from-the catheter hub 120 by a distal portion thereof. The one-or-more extension legs 122 respectively include one or more extension-leg lumens, which, in turn, correspond in number to the one-or-more catheter-hub lumens. Each extension-leg lumen of the one-or-more extension-leg lumens extends through an entirety of the extension leg from a proximal end of the extension leg to a distal end of the extension leg. When the one-or-more extension legs 122 are coupled to the catheter hub 120 as shown in FIG. 1, the one-or-more catheter-hub lumens continue through the one-or-more extension-leg lumens, respectively.

Each extension-leg connector of the one-or-more extension-leg connectors 124 is over a proximal portion of an extension leg of the one-or-more extension legs 122. For example, each extension-leg connector of the one-or-more extension-leg connectors 124 can be a Luer connector over a proximal portion of an extension leg of the one-or-more extension legs 122. Through such an extension-leg connector, a corresponding extension leg and the extension-leg lumen thereof can be connected to another medical device and a lumen thereof.

The catheter 102 can be a monoluminal catheter or a multiluminal catheter such as a diluminal catheter, a triluminal catheter, a tetraluminal catheter, a pentaluminal catheter, a hexaluminal catheter, etc. When the catheter 102 is configured as the triluminal catheter shown in FIG. 1, the catheter 102 includes a set of three lumens. The set of three lumens includes a primary lumen 132, a secondary lumen 134, and a tertiary lumen 136 formed of fluidly connected portions of three catheter-tube lumens of the catheter tube 118, three catheter-hub lumens of a trifurcated hub for the catheter hub 120, and three extension-leg lumens of three extension legs 122. The primary lumen 132 extends from an opening in a proximal end of its corresponding Luer connector to a primary-lumen opening 138 in the distal end of the first section 126 of the catheter tube 118 or the catheter tip 130 thereof, which corresponds to a distal end of the catheter 102. The secondary lumen 134 extends from an opening in a proximal end of its Luer connector to a secondary-lumen opening 140 in a side of the distal portion of the catheter tube 118. The tertiary lumen 136 extends from an opening in a proximal end of its corresponding Luer connector to a tertiary-lumen opening 142 in the side of the distal portion of the catheter tube 118 proximal of the secondary-lumen opening 140.

### Methods not forming part of the invention

Methods include a non-claimed method of inserting the catheter 102 into a blood-vessel lumen of a patient. Such a method includes one or more steps selected from a needle tract-establishing step, a guidewire-inserting step, a needle-withdrawing step, a catheter-advancing step, and an automatic stylet-withdrawing step.

The needle tract-establishing step includes establishing a needle tract from an area of skin to the blood-vessel lumen with a needle.

The guidewire-inserting step includes inserting the guidewire 106 into a needle lumen of the needle and advancing the guidewire 106 into the blood-vessel lumen.

The needle-withdrawing step includes withdrawing the needle from the needle tract leaving the guidewire 106 in place in the blood-vessel lumen.

The catheter-advancing step includes advancing the catheter 102 including the stylet 104 disposed in the primary lumen 132 thereof over the guidewire 106 with at least the proximal portion of the guidewire 106 disposed in the stylet lumen of the stylet 104 until a sufficient portion of the catheter tube 118 of the catheter 102 is disposed in the blood-vessel lumen. Again, the primary lumen 132 of the catheter 102 has the catheter inner diameter commensurate with the stylet outer diameter within at least the catheter tube 118 of the catheter 102 such that the annular space otherwise therebetween does not cause the catheter tip 130 of the catheter tube 118 to catch on the skin of the patient during the advancing of the catheter 102 into the blood-vessel lumen of the patient.

The automatic stylet-withdrawing step includes withdrawing both the guidewire 106 and the stylet 104 from the catheter 102. As set forth above with respect to FIGS. 2-5, the guidewire 106 includes the proximal portion having the first outer diameter and the distal portion including the dilated section 112 having the second outer diameter greater than the first outer diameter. Alternatively, both the proximal and distal portions of the guidewire 106 have the first outer diameter as set forth with respect to FIGS. 6-9. Accordingly, either the dilated section 112 or the spherical end cap 115 of the guidewire 106 butts against the stylet tip 116 of the stylet 104 when the guidewire 106 is proximally withdrawn from the catheter 102 for automatically withdrawing the stylet 104 from the catheter 102 with the guidewire 106.

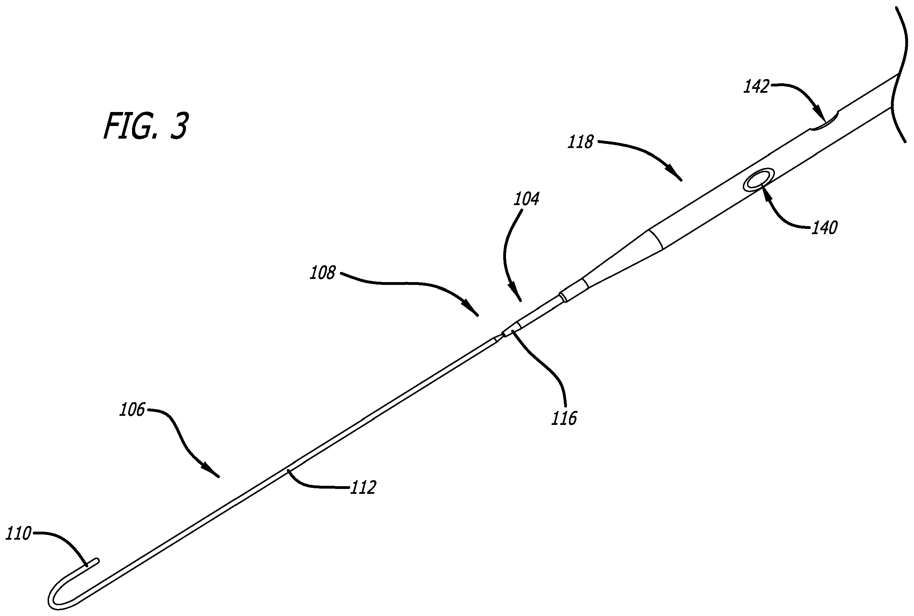

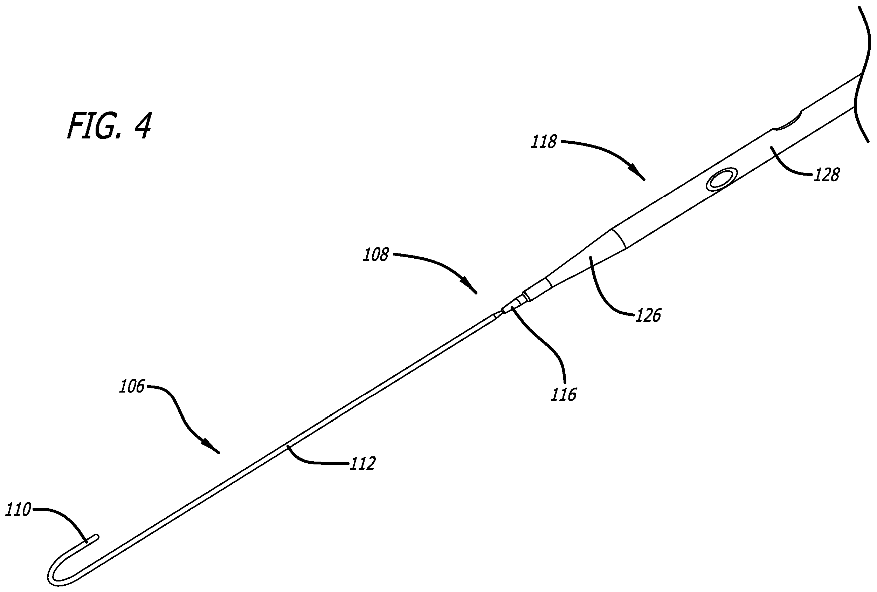

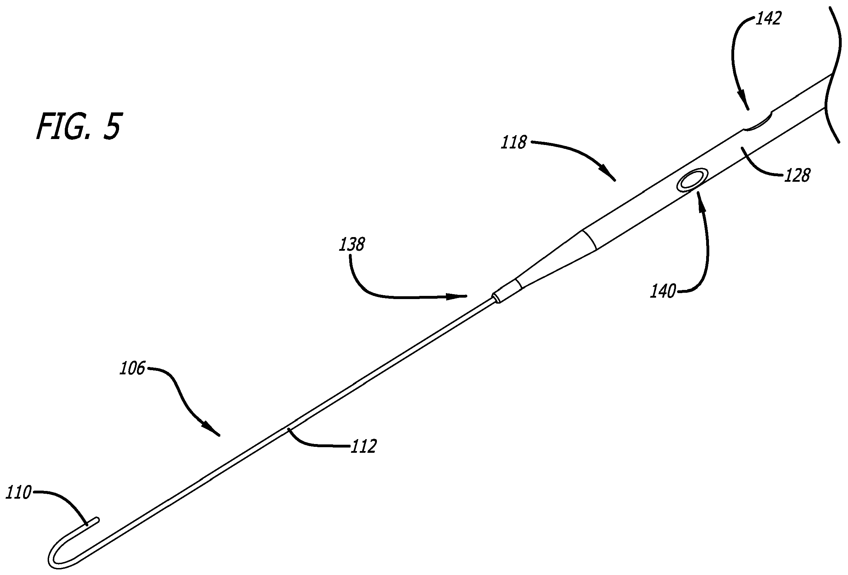

As to the automatic stylet-withdrawing step in accordance with FIGS. 2-5, the automatic stylet-withdrawing step commences with proximally withdrawing the guidewire 106 from the catheter 102 as shown in FIG. 2. With continued withdrawing of the guidewire 106 from the catheter 102 as shown in FIG. 3, the dilated section 112 of the guidewire 106 butts against the stylet tip 116 of the stylet 104. With continued withdrawing of the guidewire 106 from the catheter 102 as shown in FIG. 4, the dilated section 112 of the guidewire 106 continues to butt against the stylet tip 116 of the stylet 104 while withdrawing the guidewire 106 from the catheter 102, thereby automatically withdrawing the stylet 104 with the guidewire 106 as well. While the dilated section 112 of the guidewire 106 continues to butt against the stylet tip 116 of the stylet 104 in FIG. 5, the dilated section 112 of the guidewire 106 and the stylet tip 116 of the stylet 104 are within the primary lumen 132 of the catheter 102 until completely withdrawn in accordance the automatic stylet-withdrawing step.

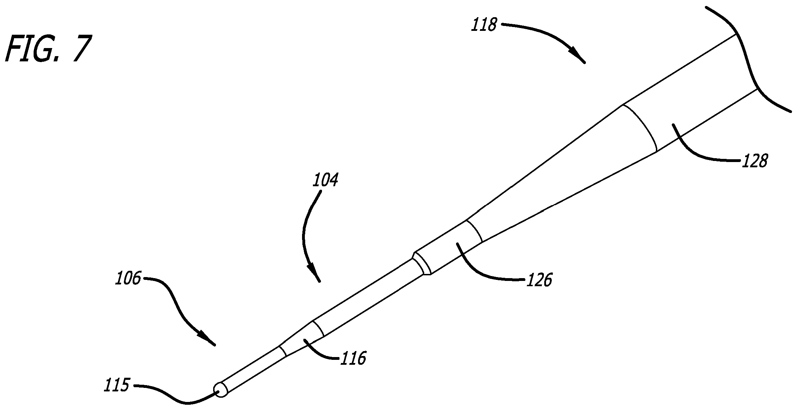

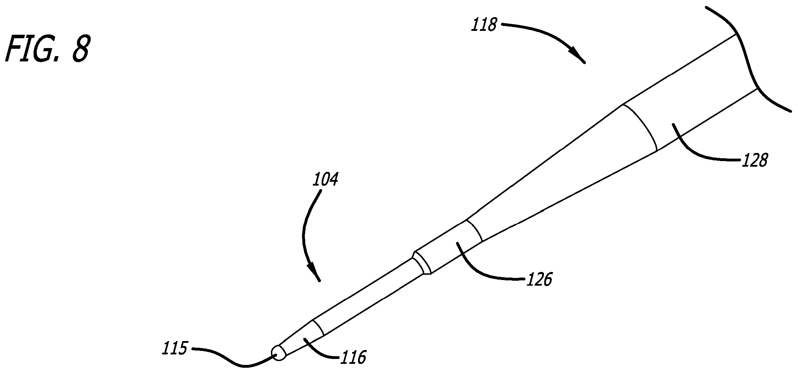

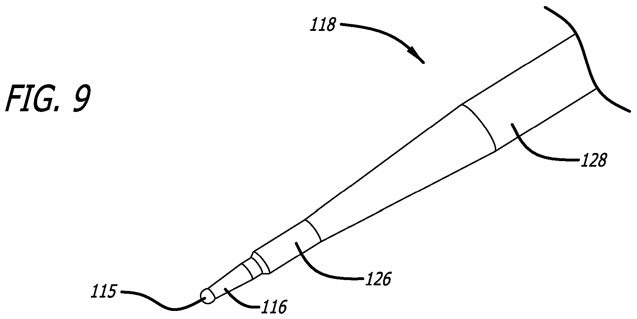

As to the automatic stylet-withdrawing step in accordance with FIGS. 6-9, the automatic stylet-withdrawing step commences with proximally withdrawing the guidewire 106 from the catheter 102 as shown in FIGS. 6 and 7. With continued withdrawing of the guidewire 106 from the catheter 102 as shown in FIG. 8, the spherical end cap 115 of the guidewire 106 butts against the stylet tip 116 of the stylet 104. With continued withdrawing of the guidewire 106 from the catheter 102 as shown in FIG. 9, the spherical end cap 115 of the guidewire 106 continues to butt against the stylet tip 116 of the stylet 104 while withdrawing the guidewire 106 from the catheter 102, thereby automatically withdrawing the stylet 104 with the guidewire 106 as well. The spherical end cap 115 of the guidewire 106 continues to butt against the stylet tip 116 of the stylet 104 within the primary lumen 132 of the catheter 102 until completely withdrawn in accordance the automatic stylet-withdrawing step.

While some particular embodiments have been disclosed herein, and while the particular embodiments have been disclosed in some detail, it is not the intention for the particular embodiments to limit the scope of the concepts provided herein. Additional adaptations or modifications can appear to those of ordinary skill in the art, and, in broader aspects, these adaptations or modifications are encompassed as well, as long as they fall under the subject-matter claimed. Accordingly, departures may be made from the particular embodiments disclosed herein without departing from the scope of the concepts provided herein, as defined in the appended claims.

## Claims

1. A stylet assembly (108), comprising:
a guidewire (106), including:
a proximal portion;
a distal portion, the proximal and distal portions of the guidewire having a first outer diameter; and
a guidewire tip (110) in the distal portion of the guidewire (106) terminating with a spherical end cap (115), the spherical end cap having a third diameter greater than the first outer diameter,
a stylet (104) including:
a stylet tip (116); and
a stylet lumen having a stylet inner diameter commensurate with the first outer diameter of the guidewire (106), at least the proximal portion of the guidewire disposed in the stylet lumen of the stylet with a corresponding orientation, and the spherical end cap (115) of the guidewire configured to butt against the stylet tip (116) when the guidewire (106) is proximally withdrawn from a catheter for automatically withdrawing the stylet (104) from the catheter (102) with the guidewire (106),
wherein the guidewire tip (110) is an atraumatic 'J'-shaped guidewire tip.

2. A stylet assembly (108), comprising:
a guidewire (106) including:
a proximal portion having a first outer diameter;
a distal portion including a dilated section (112) having a second outer diameter greater than the first outer diameter; and
a guidewire tip (110) in the distal portion of the guidewire (106); and
a stylet (104) including:
a stylet tip (106); and
a stylet lumen having a stylet inner diameter commensurate with the first outer diameter of the guidewire (106), at least the proximal portion of the guidewire disposed in the stylet lumen of the stylet with a corresponding orientation, and the dilated section (112) of the guidewire (106) configured to butt against the stylet tip (116) when the guidewire (106) is proximally withdrawn from a catheter (102) for automatically withdrawing the stylet (104) from the catheter with the guidewire (106),
wherein the guidewire tip (110) is an atraumatic 'J'-shaped guidewire tip.

3. The stylet assembly of claim 2, wherein the guidewire (106) includes a step delimiting the dilated section (112) with the second outer diameter from a remainder of the guidewire (106) with the first outer diameter.

4. The stylet assembly of claim 2, wherein the guidewire (106) includes a taper from the dilated section (112) with the second outer diameter to a remainder of the guidewire with the first outer diameter.

5. A catheter assembly, comprising:
the stylet assembly of any of claims 2 to 4; and
a catheter (102) including:
a catheter tube (118) including a catheter tip (130); and
a primary lumen (132) having a catheter inner diameter commensurate with a stylet outer diameter within at least the catheter tube (118), the stylet (104) disposed in the primary lumen (132) of the catheter with a corresponding orientation, at least the proximal portion of the guidewire (106) disposed in the stylet lumen of the stylet (104) with a corresponding orientation, and the dilated section (112) of the guidewire configured to butt against the stylet tip (116) when the guidewire is proximally withdrawn from the catheter (102) for automatically withdrawing the stylet (104) from the catheter (102) with the guidewire (106).

6. The catheter assembly of claim 5, wherein the catheter (102) is a triluminal catheter including a trifurcated catheter hub (120) coupled to a proximal portion of the catheter tube (118) and three extension legs (122) extending from the catheter hub, each extension leg of the three extension legs including a Luer connector (124) coupled to a proximal portion of the extension leg.

7. The catheter assembly of claim 6, wherein the primary lumen (132) extends from an opening in a proximal end of a first Luer connector to an opening (138) in the catheter tip (130), a secondary lumen (134) extends from an opening in a proximal end of a second Luer connector to a secondary-lumen opening (140) in a distal portion of the catheter tube (118), and a tertiary lumen (136) extends from an opening in a proximal end of a third Luer connector to a tertiary-lumen opening (142) in the distal portion of the catheter tube (118) proximal of the secondary-lumen opening (140).

## Patentansprüche

1. Stilettanordnung (108), umfassend:
einen Führungsdraht (106), einschließlich:
eines proximalen Abschnitts;
eines distalen Abschnitts, wobei der proximale und der distale Abschnitt des Führungsdrahts einen ersten Außendurchmesser aufweisen; und
einer Führungsdrahtspitze (110) im distalen Abschnitt des Führungsdrahts (106), die mit einer kugelförmigen Endkappe (115) endet, wobei die kugelförmige Endkappe einen dritten Durchmesser aufweist, der größer ist als der erste Außendurchmesser,
ein Stilett (104), einschließlich:
einer Stilettspitze (116); und
eines Stilettlumens, aufweisend einen Stilettinnendurchmesser, der dem ersten Außendurchmesser des Führungsdrahts (106) angemessen ist, wobei mindestens der proximale Abschnitt des Führungsdrahts in dem Stilettlumen des Stiletts in einer entsprechenden Orientierung angeordnet ist und die kugelförmige Endkappe (115) des Führungsdrahts ausgebildet ist, um an der Stilettspitze (116) anzuliegen, wenn der Führungsdraht (106) proximal aus einem Katheter herausgezogen wird, um das Stilett (104) automatisch mit dem Führungsdraht (106) aus dem Katheter (102) herauszuziehen,
wobei die Führungsdrahtspitze (110) eine atraumatische, "J"-förmige Führungsdrahtspitze ist.

2. Stilettanordnung (108), umfassend:
einen Führungsdraht (106) einschließlich:
eines proximalen Abschnitts, der einen ersten Außendurchmesser aufweist;
eines distalen Abschnitts, der eine erweiterte Sektion (112), die einen zweiten Außendurchmesser größer als der erste Außendurchmesser aufweist, einschließt; und
einer Führungsdrahtspitze (110) im distalen Abschnitt des Führungsdrahts (106); und
ein Stilett (104), einschließlich:
einer Stilettspitze (106); und
eines Stilettlumens, aufweisend einen Stilettinnendurchmesser, der dem ersten Außendurchmesser des Führungsdrahts (106) angemessen ist, wobei mindestens der proximale Abschnitt des Führungsdrahts in entsprechender Orientierung im Stilettlumen des Stiletts angeordnet ist, und die erweiterte Sektion (112) des Führungsdrahts (106) ausgebildet ist, um an der Stilettspitze (116) anzuliegen, wenn der Führungsdraht (106) proximal aus einem Katheter (102) herausgezogen wird, um das Stilett (104) automatisch mit dem Führungsdraht (106) aus dem Katheter herauszuziehen, wobei die Führungsdrahtspitze (110) eine atraumatische, "J"-förmige Führungsdrahtspitze ist.

3. Stilettanordnung nach Anspruch 2, wobei der Führungsdraht (106) eine Stufe einschließt, die die erweiterte Sektion (112) mit dem zweiten Außendurchmesser von einem Rest des Führungsdrahts (106) mit dem ersten Außendurchmesser abgrenzt.

4. Stilettanordnung nach Anspruch 2, wobei der Führungsdraht (106) eine Verjüngung von der erweiterten Sektion (112) mit dem zweiten Außendurchmesser zu einem Rest des Führungsdrahts mit dem ersten Außendurchmesser einschließt.

5. Katheteranordnung, umfassend:
die Stilettanordnung nach einem der Ansprüche 2 bis 4; und
einen Katheter (102), einschließlich:
eines Katheterschlauchs (118), einschließlich einer Katheterspitze (130); und
eines primären Lumens (132), aufweisend einen Katheterinnendurchmesser, der dem Stilettaußendurchmesser mindestens innerhalb des Katheterschlauchs (118) angemessen ist, wobei das Stilett (104) in entsprechender Orientierung im primären Lumen (132) des Katheters angeordnet ist, mindestens der proximale Abschnitt des Führungsdrahts (106) im Stilettlumen des Stiletts (104) in einer entsprechenden Orientierung angeordnet ist, und die erweiterte Sektion (112) des Führungsdrahtes ausgebildet ist, um an der Stilettspitze (116) anzuliegen, wenn der Führungsdraht proximal aus dem Katheter (102) herausgezogen wird, um das Stilett (104) automatisch mit dem Führungsdraht (106) aus dem Katheter (102) herauszuziehen.

6. Katheteranordnung nach Anspruch 5, wobei der Katheter (102) ein triluminaler Katheter ist, der eine dreigabelige Katheternabe (120), die mit einem proximalen Abschnitt des Katheterschlauchs (118) gekoppelt ist, und drei Verlängerungsschenkel (122) einschließt, die sich von der Katheternabe aus erstrecken, wobei jeder der drei Verlängerungsschenkel einen Luer-Verbinder (124) einschließt, der mit einem proximalen Abschnitt des jeweiligen Verlängerungsschenkels gekoppelt ist.

7. Katheteranordnung nach Anspruch 6, wobei sich das primäre Lumen (132) von einer Öffnung in einem proximalen Ende eines ersten Luer-Verbinders zu einer Öffnung (138) in der Katheterspitze (130) erstreckt, sich ein sekundäres Lumen (134) von einer Öffnung in einem proximalen Ende eines zweiten Luer-Verbinders zu einer Sekundärlumenöffnung (140) in einem distalen Abschnitt des Katheterschlauchs (118) erstreckt, und sich ein tertiäres Lumen (136) von einer Öffnung in einem proximalen Ende eines dritten Luer-Verbinders bis zu einer Tertiärlumenöffnung (142) im distalen Abschnitt des Katheterschlauchs (118) proximal zur Sekundärlumenöffnung (140) erstreckt.

## Revendications

1. Ensemble (108) formant stylet, comprenant :
un fil-guide (106), incluant :
une portion proximale ;
une portion distale, les portions proximale et distale du fil-guide ayant un premier diamètre externe ; et
une pointe (110) de fil-guide située dans la portion distale du fil-guide (106), se terminant par un embout terminal sphérique (115), l'embout terminal sphérique ayant un troisième diamètre supérieur au premier diamètre externe,
un stylet (104) incluant :
une pointe de stylet (116) ; et
une lumière de stylet ayant un diamètre interne de stylet correspondant à ce premier diamètre externe du fil-guide (106), au moins la portion proximale du fil-guide étant disposée dans la lumière de stylet du stylet avec une orientation correspondante, et l'embout terminal sphérique (115) du fil-guide étant configuré pour venir en butée contre la pointe de stylet (116) lorsque le fil-guide (106) est retiré en direction proximale d'un cathéter pour le retrait automatique du stylet (104) hors du cathéter (102) avec le fil-guide (106),
dans lequel la pointe (110) de fil-guide est une pointe de fil-guide atraumatique en forme de J.

2. Ensemble (108) formant stylet, comprenant :
un fil-guide (106) incluant :
une portion proximale, ayant un premier diamètre externe ;
une portion distale, comprenant une section dilatée (112) ayant un deuxième diamètre externe supérieur au premier diamètre externe ; et
une pointe (110) de fil-guide située dans la portion distale du fil-guide (106) ; et un stylet (104) incluant :
une pointe de stylet (106) ; et
une lumière de stylet, ayant un diamètre interne de stylet correspondant à ce premier diamètre externe du fil-guide (106), au moins la portion proximale du fil-guide étant disposée dans la lumière de stylet du stylet avec une orientation correspondante, et la section dilatée (112) du fil-guide (106) étant configurée pour venir en butée contre la pointe de stylet (116) lorsque le fil-guide (106) est retiré en direction proximale d'un cathéter (102) pour le retrait automatique du stylet (104) hors du cathéter avec le fil-guide (106), dans lequel la pointe (110) de fil-guide est une pointe de fil-guide atraumatique en forme de J.

3. Ensemble formant stylet selon la revendication 2, dans lequel le fil-guide (106) comprend un épaulement délimitant la section dilatée (112) ayant le deuxième diamètre externe à partir d'un reste du fil-guide (106) ayant le premier diamètre externe.

4. Ensemble formant stylet selon la revendication 2, dans lequel le fil-guide (106) comprend un effilement depuis la section dilatée (112) ayant le deuxième diamètre externe jusqu'à un reste du fil-guide ayant le premier diamètre externe.

5. Ensemble formant cathéter, comprenant :
l'ensemble formant stylet selon l'une quelconque des revendications 2 à 4 ; et
un cathéter (102) incluant :
un tube de cathéter (118), incluant une pointe de cathéter (130) ; et
une lumière principale (132), ayant un diamètre interne de cathéter correspondant à un diamètre externe de stylet à l'intérieur d'au moins le tube de cathéter (118), le stylet (104) étant disposé dans la lumière principale (132) du cathéter avec une orientation correspondante, au moins la portion proximale du fil-guide (106) étant disposée dans la lumière de stylet du stylet (104) avec une orientation correspondante, et la section dilatée (112) du fil-guide étant configurée pour venir en butée contre la pointe de stylet (116) lorsque le fil-guide est retiré en direction proximale du cathéter (102) pour le retrait automatique du stylet (104) hors du cathéter (102) avec le fil-guide (106).

6. Ensemble formant cathéter selon la revendication 5, dans lequel le cathéter (102) est un cathéter à triple lumière comprenant un nœud de cathéter trifurqué (120) relié à une portion proximale du tube de cathéter (118) et trois branches d'extension (122) s'étendant depuis le moyeu de cathéter, chaque branche d'extension des trois branches d'extension incluant un raccord Luer (124) relié à une portion proximale de la branche d'extension.

7. Ensemble formant cathéter selon la revendication 6, dans lequel la lumière principale (132) s'étend depuis une ouverture dans une extrémité proximale d'un premier raccord Luer jusqu'à une ouverture (138) dans la pointe de cathéter (130), une lumière secondaire (134) s'étend depuis une ouverture dans une extrémité proximale d'un deuxième raccord Luer jusqu'à une ouverture (140) de lumière secondaire dans une portion distale du tube de cathéter (118), et une lumière tertiaire (136) s'étend depuis une ouverture dans une extrémité proximale d'un troisième raccord Luer jusqu'à une ouverture (142) de lumière tertiaire dans la portion distale du tube de cathéter (118) en position proximale de l'ouverture (140) de lumière secondaire.
